# EUROPEAN PATENT SPECIFICATION

(11) **EP 1 663 367 B1**
(45) Date of publication and mention of the grant of the patent: **19.05.2010**
(21) Application number: 04768345.3
(22) Date of filing: 07.09.2004
(51) Int. Cl.: A61M 16/20

(54) **PEEP VALVE**
PEEP-VENTIL
SOUPAPE A PRESSION POSITIVE EN FIN D'EXPIRATION

(30) Priority: 11.09.2003 GB 0321266
(43) Date of publication of application: 07.06.2006
(73) Proprietor: Intersurgical AG, Vaduz (LI)
(72) Inventor: Wakefield, Adrian John, Reading RG6 1PL (GB); Jassell, Surinderjit, Windsor, Berkshire, SL4 5RQ (GB)
(74) Representative: Adamson Jones
(86) International application number: PCT/GB2004/003798
(87) International publication number: WO 2005/025659

(56) References cited:
- DE-A- 10 046 872
- US-A- 4 257 453
- US-A- 4 428 392
- US-A- 4 823 828
- US-A- 5 002 050

## Description

This invention relates to valves that apply Positive End Expiratory Pressure (PEEP) to an artificial respiratory circuit, ie so-called PEEP valves, and in particular to PEEP valves that allow bi-directional flow.

When a patient is unable to breathe unaided, or requires assistance with breathing, the patient is usually connected to an artificial respiratory circuit including a ventilator programmed by a clinician to deliver an appropriate volume of air, or an air/oxygen mixture, to the patient. In such a respiratory circuit, where the patient is breathing spontaneously but requiring pressure support, it is desirable to prevent the patient from exhaling fully, and therefore the patient's lungs from deflating fully. This is because complete deflation, and subsequent reflation, of the patient's lungs requires a significant amount of the patient's energy.

Prevention of total exhalation is generally achieved by including a mechanism in the respiratory circuit which only allows exhaled breath above an appropriate exhalation pressure to escape the respiratory circuit. Prevention of total exhalation in this way is known as applying "PEEP" to the respiratory circuit, where "PEEP" refers to Positive End Expiratory Pressure. A widely used method of applying PEEP to a respiratory circuit is by using a so-called PEEP valve to provide a fixed and pre-determined release pressure to the respiratory circuit.

PEEP valves typically comprise an inlet port for connection to a respiratory circuit, an outlet port for venting the exhaled breath, and a valve mechanism controlling the flow of exhaled breath between the inlet port and the outlet port. A widely used valve mechanism comprises a sealing disc that is spring biased into engagement with an annular valve seat so that exhaled breath at a pressure above a pre-determined exhalation pressure can displace the sealing disc from the valve seat and the air can therefore pass from the inlet port to the outlet port, but air cannot pass from the outlet port to the inlet port. Such PEEP valves thus allow flow of air through the valve in only a single direction.

PEEP valves are also known that allow the bi-directional flow of air through the valve. Such a construction allows air to be drawn into the respiratory circuit through the PEEP valve. This may be necessary if, for example, the patient requires more air than is being supplied by the ventilator or if the ventilator flow ceases. PEEP valves that allow the bi-directional flow of air through the valve may therefore be safer than conventional unidirectional PEEP valves.

Currently available bi-directional PEEP valves comprise a first sealing disc that is spring biased into engagement with a valve seat and has a plurality of apertures, and a second flexible sealing disc fixed to the first sealing disc at its centre so that the second sealing disc overlies the first sealing disc and occludes the plurality of apertures. An example of this type of bi-directional PEEP valve is disclosed by US 4,823,828. In this way, exhaled breath at a pressure above a pre-determined exhalation pressure can pass from the inlet port to the outlet port, as described above in relation to unidirectional PEEP valves, and air drawn through the outlet port at a pressure sufficient to deform the second sealing disc into a cone-like shape can pass from the outlet port to the inlet port.

A disadvantage of such bidirectional PEEP valves is that the resistance to air flow from the outlet port to the inlet port may be relatively high, particularly at low flow rates, and this may inhibit the operation of the valve.

There has now been devised an improved PEEP valve which overcomes or substantially mitigates the above-mentioned and/or other disadvantages associated with the prior art.

According to the invention, there is provided a PEEP valve for use in a respiratory circuit, the valve comprising a housing having an inlet port in fluid communication with an outlet port, a valve seat situated between the inlet port and the outlet port, and a sealing disc movably mounted within the housing and resiliently biased into engagement with the valve seat such that a predetermined positive pressure differential between the inlet and outlet ports, in use, causes the sealing disc to disengage from the valve seat and allow flow of gas from the inlet port to the outlet port, the sealing disc including at least one aperture and there being provided a closure of flexible material that occludes the aperture, **characterised in that** the closure is clamped to the sealing disc by a retainer such that a negative pressure differential between the inlet and outlet ports, in use, causes the closure to hinge about a substantially linear fold that forms in the closure, thereby exposing the aperture and allowing gas to flow from the outlet port to the inlet port.

The closure of the PEEP valve according to the invention is adapted to hinge about a linear fold that forms in the closure in response to a negative pressure differential between the inlet and outlet ports. In contrast, the closure of a conventional bi-directional PEEP valve comprises a circular disc that is fixed to the sealing disc at its centre and deforms into a cone-like shape in response to a negative pressure differential between the inlet and outlet ports. The PEEP valve according to the invention is advantageous principally in that a lower negative pressure differential between the inlet and outlet ports is required to deform the closure, for a given orifice size, than for conventional bi-directional PEEP valves. The PEEP valve according to the invention therefore has a lower resistance to air flow from the outlet port to the inlet port than is the case for conventional valves.

The housing of the PEEP valve according to the invention preferably defines a valve chamber between the outlet port and the valve seat. The sealing disc is most preferably mounted within the valve chamber. In particular, the valve chamber is preferably substantially cylindrical with the inlet port at one end of the valve chamber, the other end of the valve chamber being closed, and the outlet port branching perpendicularly from the side of the valve chamber. In this case, the closed end of the valve chamber preferably includes a mounting for the sealing disc. Most preferably, at least part of the mounting is formed integrally with the closed end of the valve chamber. Most preferably, the closed end of the valve chamber is formed by a closure that is formed integrally with at least part of the mounting for the sealing disc and which is closely received within the housing.

The mounting for the sealing disc preferably comprises a channel of constant cross-section that has an open end for receiving a shaft so that the shaft is able to move reciprocally within the channel. The end of the shaft that projects from the open end of the channel is preferably fixed to the sealing disc. Most preferably, the shaft has a rod extending co-axially from the end of the shaft which is fixed within a central bore of the sealing disc.

The valve preferably includes resilient means for biasing the sealing disc into engagement with the valve seat. The resilient means is preferably a spring and is most preferably a constant force spring. Preferably, the spring is mounted on the mounting for the sealing disc, and preferably acts on the end of the shaft remote from the sealing disc.

The valve seat is preferably a raised formation on the interior surface of the housing. Most preferably, the valve seat is generally annular in form and defines a central opening. The sealing disc preferably engages the valve seat so as to seal the opening defined by the valve seat. The valve seat is preferably annular in shape, and the sealing disc is preferably circular in shape.

The sealing disc preferably includes two or more apertures, and most preferably six or more apertures. The PEEP valve may therefore include two or more closures, each closure occluding one or more apertures. In preferred embodiments, the apertures are arranged in groups, each group of apertures cooperating with one of two or more closures. This arrangement is advantageous because a closure occluding a group of two or more apertures is much less likely to be drawn through those apertures by high flow rates from the inlet port to the outlet port than a closure occluding a single, larger aperture.

In a preferred embodiment, the sealing disc is provided with two diametrically opposed groups of apertures, and each group of apertures is occluded by a single closure, such that the PEEP valve has a pair of diametrically opposed closures. Most preferably, each of these closures is generally elliptical in shape.

The closure(s) preferably overlie the surface of the sealing disc that faces in the direction of the inlet port. Each closure may overlie a raised formation that extends about the perimeter of the aperture, or apertures, occluded by that closure. In this case, each closure preferably overlies an upper edge of the raised formation.

Where the sealing disc includes two or more closures, each closure may be a distinct component fixed to the sealing disc. Most preferably, however, the closures of the PEEP valve all form part of a single closure component that is fixed to the sealing disc. Each closure, or the closure component, is preferably formed in an elastomeric material, such as silicone rubber, and is most preferably formed from a sheet of silicone rubber.

In a preferred embodiment, the retainer has a central bore for engagement with the shaft of the sealing disc mounting. The retainer preferably engages the shaft and captivates the closure component in close abutment with the sealing disc.

The linear fold in each closure, which is caused by the negative pressure differential between the inlet and outlet ports, will form across the closure, normally adjacent the region of the closure that is fixed to the sealing disc.

Each closure is therefore preferably fixed to the sealing disc at one edge of the closure and beyond the periphery of the aperture, or apertures, that the closure occludes. To facilitate the formation of a linear fold, each closure may be fixed, and most preferably clamped, by a retainer with a straight edge adjacent to the closure about which the linear fold can form.

The invention will now be described in greater detail, by way of illustration only, with reference to the accompanying drawings, in which
Figure 1 is a side view of a first embodiment of a PEEP valve according to the invention;
Figure 2 is a cross-sectional view of the PEEP valve of Figure 1;
Figure 3a is a view similar to that of Figure 2 of the PEEP valve during exhalation through the valve;
Figure 3b is a view similar to that of Figure 2 of the PEEP valve during inhalation through the valve;
Figure 4a is a perspective view of the front face of a sealing disc, which forms part of the first embodiment of a PEEP valve according to the invention, at rest;
Figure 4b is a perspective view of the rear face of the sealing disc of the first embodiment at rest;
Figure 5a is a perspective view of the front face of the sealing disc of the first embodiment during inhalation through the valve;
Figure 5b is a perspective view of the rear face of the sealing disc of the first embodiment during inhalation through the valve;
Figure 6 is a frontal view, showing hidden detail, of a sealing disc that forms part of a second embodiment of a PEEP valve according to the invention;
Figure 7a is a perspective view of the front face of the sealing disc of the second embodiment at rest;
Figure 7b is a perspective view of the rear face of the sealing disc of the second embodiment at rest;
Figure 7c is a cross-sectional view, along the line VII-VII in Figure 6, of the sealing disc of the second embodiment at rest;
Figure 8a is a perspective view of the front face of the sealing disc of the second embodiment during inhalation through the valve;
Figure 8b is a perspective view of the rear face of the sealing disc of the second embodiment during inhalation through the valve; and
Figure 8c is a cross-sectional view, along the line VII-VII in Figure 6, of the sealing disc of the second embodiment during inhalation through the valve.

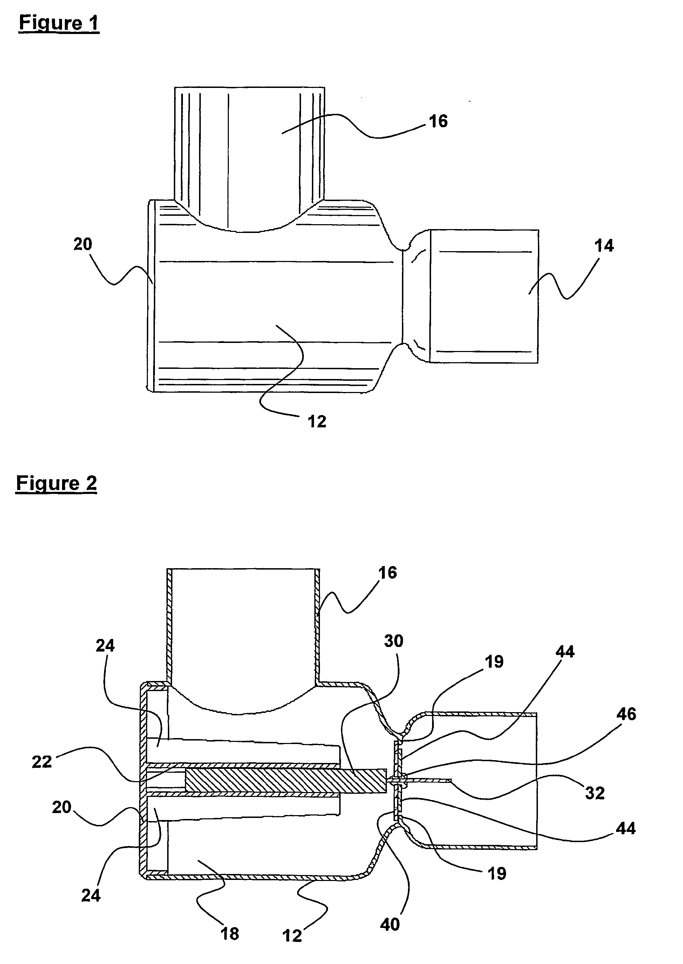

Figures 1 and 2 show a first embodiment of the PEEP valve according to the invention. The PEEP valve comprises a cylindrical housing 12, which has an inlet port 14 formed at one end of the housing 12 and is sealed by a closure 20 at the other end, and an outlet port 16 that branches perpendicularly from the side wall of the housing 12. The housing 12 and closure 20 together define a valve chamber 18, and the housing 12 and inlet port 14 are connected by a neck portion that has an annular valve seat 19 extending inwardly from its interior surface. The housing 12, inlet port 14 and outlet port 16 are formed integrally by injection moulding a suitable plastics material. The closure 20 is formed separately, also by injection moulding a suitable plastics material.

The closure 20 comprises a circular disc, and an annular skirt extending perpendicularly from the disc. The annular skirt is closely received within the end of the housing 12 opposite the inlet port 14 so that the circular disc abuts that end of the housing 12 and forms an end wall of the valve chamber 18.

A guide formation 22 is formed integrally with the closure 20 and extends from the inwardly facing surface of the closure 20 towards the inlet port 14. The guide formation 22 comprises a central channel of rectangular cross-section and a plurality of support struts 24. The central channel of the guide formation 22 has an open end adjacent to the inlet port 14 and receives a shaft 30 of rectangular cross-section so that the shaft 30 is able to move reciprocally within the channel. A constant force spring (not visible in Figure 2) is mounted on the guide formation 22 and biases the shaft 30 towards the valve seat 19 at a constant force.

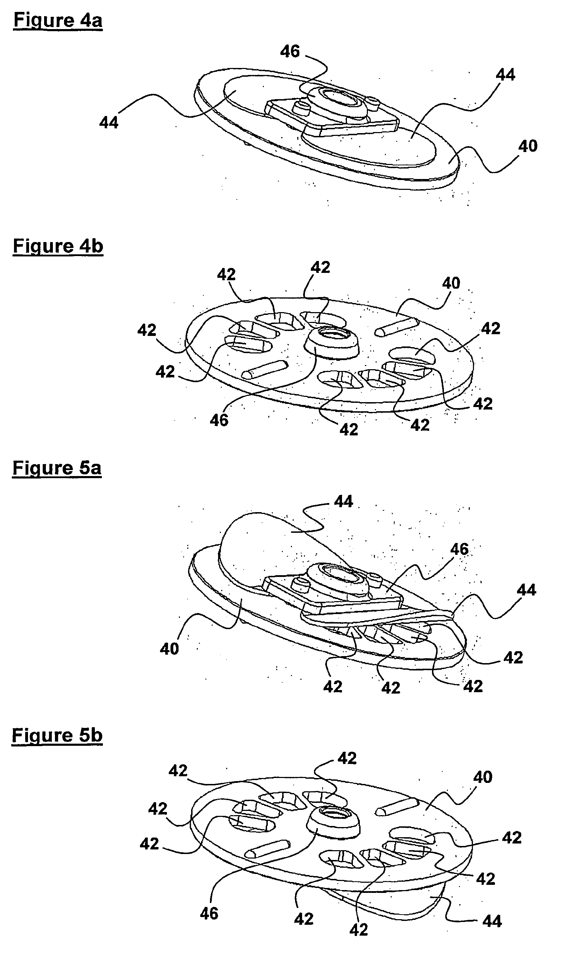

A cylindrical rod 32 of smaller cross-section than the shaft 30 extends co-axially from the end of the shaft 30 that is adjacent to the valve seat 19. The cylindrical rod 32 is secured within a central bore of a sealing disc 40 so that the sealing disc 40 is biased into engagement with the valve seat 19. In this embodiment, the sealing disc 40 has two groups of apertures, each group of apertures being occluded by a closure of flexible material 44, as shown more clearly in Figures 4a, 4b, 5a and 5b. The closures of flexible material 44 are attached to the centre of the sealing disc by a retainer 46. The retainer 46 is fastened within the central bore of the sealing disc 40 so that an edge of each closure 44 is clamped to the sealing disc 40. The retainer 46 has a central bore within which the cylindrical rod 32 is fixed. The sealing disc 40, closures of flexible material 44, and retainer 46 are described more fully below in relation to Figures 4a, 4b, 5a and 5b.

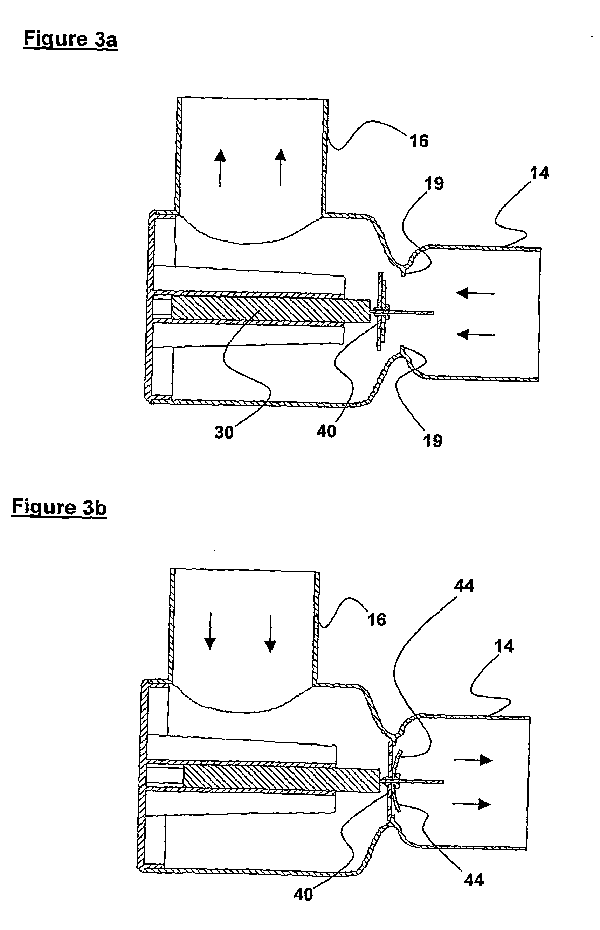

In use, the inlet port 14 of the PEEP valve is connected to an artificial respiratory circuit such that the exhaled breath of a patient is supplied to the inlet port 14. As shown in Figure 3a, exhaled breath at the inlet port 14 above a pre-determined expiration pressure, which is determined by the constant force spring, will cause the shaft 30 and sealing disc 40 to move away from the valve seat 19. Exhaled breath will then exit the PEEP valve through the outlet port 16.

In the event that it is necessary for the patient to draw air through the PEEP valve from the outlet port 16 to the inlet port 14, the patient will lower the air pressure at the inlet port 14. As shown in Figure 3b, this will cause the closures of flexible material 44 to deform away from the sealing disc 40 and the two groups of apertures, and air to be drawn from the outlet port 16 to the inlet port 14.

The sealing disc 40, closures of flexible material 44 and retainer 46 are all shown more clearly in Figures 4a, 4b, 5a and 5b.

Figures 4a and 4b show the sealing disc 40, closures of flexible material 44 and retainer 46 at rest. The sealing disc 40 is circular in shape and is formed in a relatively rigid plastics material so that the sealing disc 40 retains its shape during normal use of the PEEP valve. The sealing disc 40 has a central bore and two diametrically opposed groups of four apertures 42. Each group of apertures 42 is arranged along a curved line that approximately follows the curvature of the peripheral edge of the sealing disc 40.

The closures of flexible material 44 all form part of a single elastomeric component that overlies the front surface of the sealing disc 40. The elastomeric component comprises the pair of closures 44 joined by a narrow central bridge. The central bridge has a central bore in which the retainer 46 in received. The closures of flexible material 44 are generally elliptical in shape and occlude the two groups of apertures 42 completely when at rest. In this embodiment, the elastomeric component is formed from a sheet of silicone rubber.

The retainer 46 has the form of a stud with a rectangular head portion, a relatively short shaft, and a central bore. The central bore is tapered towards the tip of the shaft. The shaft of the retainer 46 may be urged through the central bores of the elastomeric component and the sealing disc 40 so that the retainer 46 engages the sealing disc 40 with a snap fit, the head portion clamping the central bridge of the elastomeric component to the front surface of the sealing disc 40. The head portion of the retainer 46 includes a straight edge adjacent to each closure 44 about which the closure 44 can fold during use.

The closures of flexible material 44 are therefore arranged such that a greater pressure at the rear surface of the sealing disc 40 than at the front surface of the sealing disc 40 will cause each closure 44 to deform about a linear fold which forms adjacent to the retainer 46. Deformation of the closures 44 in this way allows respiratory gases to flow through the apertures 42 and hence allows respiratory gases to pass through the sealing disc 40 from the outlet port 16 to the inlet port 14. Such deformation is shown clearly in Figures 5a and 5b.

The linear fold will form to one side of, and parallel to, the major axis of the elliptical closure 44, and adjacent to the retainer 46. A lower pressure differential is required to deform a closure 44 about a linear fold than is required to deform a disc-shaped closure, which is secured to a sealing disc at its centre, into a cone-like shape as for conventional bi-directional flow PEEP valves.

The following table sets out the results of a test of the pressure resistance to air flow from the outlet valve to the inlet valve of a PEEP valve incorporating a conventional circular deformable disc that deforms into a cone-like shape ("Disc Resistance"), and a PEEP valve similar to the first embodiment of the invention described above having closures that deform about a linear fold ("Butterfly Vent Resistance").

**Table 1**

| Flow (I/m) | Disc Resistance (cmH₂O) | Butterfly Vent Resistance (cmH₂O) | Reduction in Resistance achieved by changing to the Butterfly Vent (%) |
|---|---|---|---|
| 5 | 0.21 | 0.19 | 14 |
| 10 | 0.61 | 0.40 | 34 |
| 20 | 1.65 | 0.75 | 55 |
| 40 | 3.40 | 1.60 | 53 |
| 60 | 5.19 | 2.64 | 49 |
| 80 | 7.04 | 3.90 | 45 |
| 100 | 9.24 | 5.55 | 40 |
| 120 | 11.8 | 7.20 | 39 |

Clearly, the PEEP valve according to the invention has a lower resistance to air flow through the sealing disc 40 from the outlet port 16 to the inlet port 14.

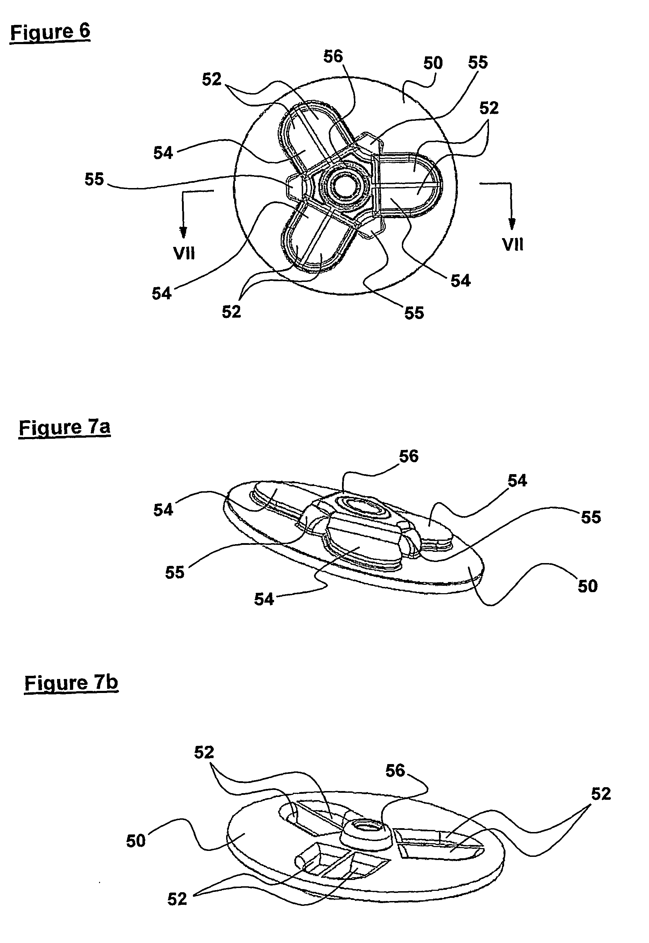

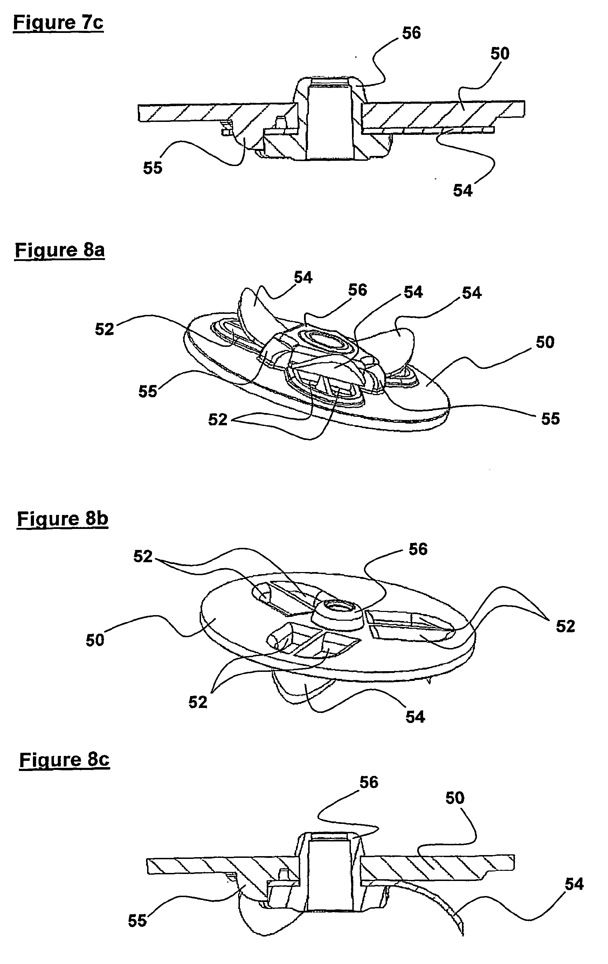

Figures 6, 7a, 7b and 7c show a sealing disc 50 that forms part of a second embodiment of the PEEP valve according to the invention. The second embodiment of the PEEP valve according to the invention is identical to the first embodiment save for the form and arrangement of the sealing disc 50, closures of flexible material 54, and retainer 56.

The two groups of apertures 42 of the first embodiment are replaced in the second embodiment by three pairs of apertures 52 arranged around the central bore of the sealing disc 50. Each pair of apertures 52 is formed by an opening having a rectangular inner portion and a semi-circular outer portion, and a radially extending divider which divides the opening into a pair of apertures 52. The front surface of the sealing disc 50 (ie the surface over which the closures of flexible material 54 lie) has a raised knife-edge formation that extends around the periphery of each aperture 52.

The pair of elliptical closures of flexible material 44 of the first embodiment are replaced in the second embodiment by three closures of flexible material 54 which each have a shape similar to, and dimensions slightly larger than, each pair of apertures 52. The three closures of flexible material all form part of a single elastomeric component that overlies the raised knife-edges of the front surface of the sealing disc 50, at rest, such that each closure of flexible material 54 occludes a pair of apertures 52 completely. The elastomeric component comprises a central portion from which the three closures 54 extend outwardly. The central portion has a central bore which receives the retainer 56. The elastomeric component is most preferably formed from a sheet of silicone rubber.

The retainer 56 of the second embodiment is identical to that of the first embodiment save that the head portion of the retainer 56 is generally triangular in shape. Each corner of the triangular head portion has a cut-away portion that receives a raised formation 55 on the front surface of the sealing disc 50. The three straight lower edges of the triangular retainer head portion are arranged along the inner straight edges of the closures 54 and the apertures 52, thereby forming edges about which the closures 54 can fold.

As for the first embodiment, the closures of flexible material 54 of the second embodiment are arranged such that a greater pressure at the rear surface of the sealing disc 50 than at the front surface of the sealing disc 50 will cause each closure 54 to deform about a linear fold which forms along a straight lower edge of the triangular head portion of the retainer 56. Deformation of the closures 54 in this way opens the apertures 52 and allows air to pass through the sealing disc 50 from the outlet port to the inlet port of the second embodiment. Such deformation is shown clearly in Figures 8a, 8b and 8c.

## Claims

1. A PEEP valve for use in a respiratory circuit, the valve comprising a housing (12) having an inlet port (14) in fluid communication with an outlet port (16), a valve seat (19) situated between the inlet port (14) and the outlet port (16), and a sealing disc (40,50) movably mounted within the housing (12) and resiliently biased into engagement with the valve seat (19) such that a predetermined positive pressure differential between the inlet and outlet ports (14,16), in use, causes the sealing disc (40,50) to disengage from the valve seat (19) and allow flow of gas from the inlet port (14) to the outlet port (16), the sealing disc (40,50) including at least one aperture (42,52) and there being provided a closure (44,54) of flexible material that occludes the aperture,
**characterised in that** the closure (44,54) is clamped to the sealing disc (40,50) by a retainer (46,56) such that a negative pressure differential between the inlet and outlet ports (14,16), in use, causes the closure (44,54) to hinge about a substantially linear fold that forms in the closure (44,54), thereby exposing the aperture and allowing gas to flow from the outlet port (16) to the inlet port (14).

2. A PEEP valve as claimed in Claim 1, wherein the retainer (46,56) includes a straight edge adjacent to the closure (44,54) about which the substantially linear fold can form.

3. A PEEP valve as claimed in Claim 1 or Claim 2, wherein the housing (12) defines a valve chamber (18) between the outlet port (16) and the valve seat (19), and the sealing disc (40,50) is mounted within the valve chamber (18).

4. A PEEP valve as claimed in Claim 3, wherein the valve chamber (18) is substantially cylindrical with the inlet port (14) at one end of the valve chamber (18), the other end of the valve chamber (18) being closed, and the outlet port (16) branching perpendicularly from the side of the valve chamber (18).

5. A PEEP valve as claimed in Claim 4, wherein the closed end of the valve chamber (18) includes a mounting for the sealing disc (40,50).

6. A PEEP valve as claimed in Claim 5, wherein the closed end of the valve chamber (18) is formed by a closure (20) that is formed integrally with at least part of the mounting for the sealing disc (40,50) and which is closely received within the housing (12).

7. A PEEP valve as claimed in any preceding claim, wherein the mounting for the sealing disc (40,50) comprises a channel of constant cross-section that has an open end for receiving a shaft (30) so that the shaft (30) is able to move reciprocally within the channel, the end of the shaft (30) that projects from the open end of the channel being fixed to the sealing disc (40,50).

8. A PEEP valve as claimed in Claim 7, wherein the shaft (30) has a rod (32) extending co-axially from the end of the shaft (30) which is fixed within a central bore of the sealing disc (40,50).

9. A PEEP valve as claimed in any preceding claim, wherein the valve includes resilient means for biasing the sealing disc (40,50) into engagement with the valve seat (19).

10. A PEEP valve as claimed in Claim 9, wherein the resilient means is a constant force spring.

11. A PEEP valve as claimed in any preceding claim, wherein the valve seat (19) is a raised formation on the interior surface of the housing (12).

12. A PEEP valve as claimed in Claim 11, wherein the valve seat (19) is generally annular in form and defines a central opening, and the sealing disc (40,50) engages the valve seat (19) so as to seal the opening defined by the valve seat (19).

13. A PEEP valve as claimed in any preceding claim, wherein the closure (44,54) overlies the surface of the sealing disc (40,50) that faces in the direction of the inlet port (14).

14. A PEEP valve as claimed in any preceding claim, wherein the closure (44,54) is formed in an elastomeric material.

15. A PEEP valve as claimed in any preceding claim, wherein the closure (44,54) is fixed to the sealing disc (40,50) at one edge of the closure (44,54) and beyond the periphery of the aperture that the closure (44,54) occludes.

16. A PEEP valve as claimed in any preceding claim, wherein the sealing disc (40,50) includes two or more apertures (42,52).

17. A PEEP valve as claimed in Claim 16, wherein the sealing disc (40,50) includes six or more apertures (42,52).

18. A PEEP valve as claimed in Claim 16 or Claim 17, wherein the apertures (42,52) are arranged in groups, each group of apertures (42,52) cooperating with one of two or more closures (44,54).

19. A PEEP valve as claimed in Claim 18, wherein each closure (44,54) occludes two or more apertures (42,52).

20. A PEEP valve as claimed in Claim 19, wherein the sealing disc (40,50) is provided with two diametrically opposed groups of apertures (42,52), and each group of apertures (42,52) is occluded by a single closure (44,54), such that the PEEP valve has a pair of diametrically opposed closures (44,54).

21. A PEEP valve as claimed in Claim 20, wherein each of the diametrically opposed closures (44,54) is generally elliptical in shape.

22. A PEEP valve as claimed in any one of Claims 18 to 21, wherein the two or more closures (44,54) form part of a single closure component that is fixed to the sealing disc (40,50).

## Patentansprüche

1. PEEP-Ventil zur Verwendung in einem Atemwegskreislauf, wobei das Ventil ein Gehäuse (12), das eine Einlassöffnung (14), die in Fluidverbindung mit einer Auslassöffnung (16) steht, einen Ventilsitz (19), der sich zwischen der Einlassöffnung (14) und der Auslassöffnung (16) befindet, und eine Dichtscheibe (40, 50) aufweist, die beweglich im Gehäuse (12) angebracht ist und derart federnd in Eingriff mit dem Ventilsitz (19) vorgespannt ist, dass in Verwendung eine vorbestimmte positive Druckdifferenz zwischen der Einlass- und der Auslassöffnung (14, 16) bewirkt, dass die Dichtscheibe (40, 50) außer Eingriff mit dem Ventilsitz (19) kommt und das Strömen von Gas von der Einlassöffnung (14) zur Auslassöffnung (16) ermöglicht wird, wobei die Dichtscheibe (40, 50) zumindest eine Öffnung (42, 52) aufweist und ein Verschluss (44, 54) aus flexiblem Material bereitgestellt ist, der die Öffnung verschließt,
**dadurch gekennzeichnet, dass** der Verschluss (44, 54) mit einem Haltestück (46, 56) derart an der Dichtscheibe (40, 50) festgeklemmt ist, dass in Verwendung eine negative Druckdifferenz zwischen der Einlass- und der Auslassöffnung (14, 16) bewirkt, dass der Verschluss (44, 54) um einen im Wesentlichen linearen Falz, der im Verschluss (44, 54) ausgebildet ist, umklappt, wodurch die Öffnung freigegeben wird und Gas von der Auslassöffnung (16) zur Einlassöffnung (14) strömen kann.

2. PEEP-Ventil nach Anspruch 1, wobei das Haltestück (46, 56) eine zum Verschluss (44, 54) benachbarte gerade Kante aufweist, um die sich der im Wesentlichen lineare Falz bilden kann.

3. PEEP-Ventil nach Anspruch 1 oder Anspruch 2, wobei das Gehäuse (12) einen Ventilsitz (18) zwischen der Auslassöffnung (16) und dem Ventilsitz (19) definiert, und die Dichtscheibe (40, 50) in der Ventilkammer (18) angebracht ist.

4. PEEP-Ventil nach Anspruch 3, wobei die Ventilkammer (18) im Wesentlichen zylinderförmig ist, wobei sich die Einlassöffnung (14) an einem Ende der Ventilkammer (18) befindet, und wobei das andere Ende der Ventilkammer (18) geschlossen ist, und wobei die Auslassöffnung (16) senkrecht von der Seite der Ventilkammer (18) abzweigt.

5. PEEP-Ventil nach Anspruch 4, wobei das geschlossene Ende der Ventilkammer (18) eine Halterung für die Dichtscheibe (40, 50) aufweist.

6. PEEP-Ventil nach Anspruch 5, wobei das geschlossene Ende der Ventilkammer (18) von einem Verschluss (20) ausgebildet wird, der einstückig mit zumindest einem Teil der Halterung für die Dichtscheibe (40, 50) ausgebildet ist und der im Gehäuse (12) eng umschlossen aufgenommen wird.

7. PEEP-Ventil nach einem der vorhergehenden Ansprüche, wobei die Halterung für die Dichtscheibe (40, 50) einen Kanal mit konstantem Querschnitt aufweist, der ein offenes Ende zur Aufnahme eines Schaftes (30) aufweist, so dass sich der Schaft (30) im Kanal hin- und herbewegen kann, wobei das Ende des Schaftes (30), das vom offenen Ende des Kanals hervorragt, an der Dichtscheibe (40, 50) befestigt ist.

8. PEEP-Ventil nach Anspruch 7, wobei der Schaft (30) einen sich koaxial vom Ende des Schaftes (30) erstreckenden Stab (32) aufweist, der in einer Mittelbohrung der Dichtscheibe (40, 50) befestigt ist.

9. PEEP-Ventil nach einem der vorhergehenden Ansprüche, wobei das Ventil Federmittel zum Vorspannen der Dichtscheibe (40, 50) in Eingriff mit dem Ventilsitz (19) aufweist.

10. PEEP-Ventil nach Anspruch 9, wobei das Federmittel eine Konstantkraftfeder ist.

11. PEEP-Ventil nach einem der vorhergehenden Ansprüche, wobei der Ventilsitz (19) eine erhöhte Ausbildung auf der Innenfläche des Gehäuses (12) ist.

12. PEEP-Ventil nach Anspruch 11, wobei der Ventilsitz (19) allgemein ringförmig ausgebildet ist und eine mittlere Öffnung definiert, und wobei die Dichtscheibe (40, 50) mit dem Ventilsitz (19) in Eingriff kommt, so dass die vom Ventilsitz (19) definierte Öffnung dicht verschlossen wird.

13. PEEP-Ventil nach einem der vorhergehenden Ansprüche, wobei der Verschluss (44, 54) auf der Oberfläche der Dichtscheibe (40, 50), die der Richtung der Einlassöffnung (14) zugewandt ist, aufliegt.

14. PEEP-Ventil nach einem der vorhergehenden Ansprüche, wobei der Verschluss (44, 54) aus elastomerem Material ausgebildet ist.

15. PEEP-Ventil nach einem der vorhergehenden Ansprüche, wobei der Verschluss (44, 54) an einem Rand des Verschlusses (44, 54) und über den Umfang der Öffnung, die der Verschluss (44, 54) verschließt, hinaus an der Dichtscheibe (40, 50) befestigt ist.

16. PEEP-Ventil nach einem der vorhergehenden Ansprüche, wobei die Dichtscheibe (40, 50) zwei oder mehr Öffnungen (42, 52) aufweist.

17. PEEP-Ventil nach Anspruch 16, wobei die Dichtscheibe (40, 50) sechs oder mehr Öffnungen (42, 52) aufweist.

18. PEEP-Ventil nach Anspruch 16 oder Anspruch 17, wobei die Öffnungen (42, 52) in Gruppen angeordnet sind, wobei jede Gruppe von Öffnungen (42, 52) mit einem von zwei oder mehr Verschlüssen (44, 54) zusammenwirkt.

19. PEEP-Ventil nach Anspruch 18, wobei jeder Verschluss (44, 54) zwei oder mehr Öffnungen (42, 52) verschließt.

20. PEEP-Ventil nach Anspruch 19, wobei die Dichtscheibe (40, 50) mit zwei einander diametral gegenüberliegenden Gruppen von Öffnungen (42, 52) bereitgestellt wird, und wobei jede Gruppe von Öffnungen (42, 52) von einem einzigen Verschluss (44, 54) verschlossen wird, derart, dass das PEEP-Ventil ein Paar einander diametral gegenüberliegender Verschlüsse (44, 54) aufweist.

21. PEEP-Ventil nach Anspruch 20, wobei jeder der einander diametral gegenüberliegenden Verschlüsse (44, 54) allgemein eine elliptische Form aufweist.

22. PEEP-Ventil nach einem der Ansprüche 18 bis 21, wobei die zwei oder mehr Verschlüsse (44, 54) Teil eines einzigen Verschlussbauteils sind, das an der Dichtscheibe (40, 50) befestigt ist.

## Revendications

1. Valve PEEP pour utilisation dans un circuit respiratoire, la valve comprenant un boîtier (12) ayant un orifice d'entrée (14) en communication fluide avec un orifice de sortie (16), un siège de valve (19) situé entre l'orifice d'entrée (14) et l'orifice de sortie (16), et un disque d'étanchéité (40, 50) monté de manière mobile à l'intérieur du boîtier (12) et sollicité de manière élastique en une mise en prise avec le siège de valve (19) de telle manière à ce qu'un différentiel de pression positif prédéterminé entre les orifices d'entrée et de sortie (14, 16), lors de l'utilisation, fasse que le disc d'étanchéité (40, 50) se libère du siège de valve (19) et permette l'écoulement de gaz de l'orifice d'entrée (14) à l'orifice de sortie (16), le disque d'étanchéité (40, 50) incluant au moins une ouverture (42, 52) et une fermeture (44, 54) en matériau flexible qui occlut l'ouverture y étant prévue,
**caractérisée en ce que** la fermeture (44, 54) est fixée au disque d'étanchéité (40, 50) par un dispositif de retenue (46, 56) de telle manière à ce qu'un différentiel de pression négatif entre les orifices d'entrée et de sortie (14, 16), lors de l'utilisation, fasse que la fermeture (44, 54) s'articule autour d'un pli essentiellement linéaire qui se forme dans la fermeture (44, 54), exposant ainsi l'ouverture et permettant au gaz de s'écouler de l'orifice de sortie (16) à l'orifice d'entrée (14).

2. Valve PEEP telle que revendiquée à la Revendication 1, où le dispositif de retenue (46, 56) inclut un bord droit adjacent à la fermeture (44, 54) autour de laquelle le pli essentiellement linéaire peut se former.

3. Valve PEEP telle que revendiquée à la Revendication 1 ou Revendication 2, où le boîtier (12) définit une chambre de valve (18) entre l'orifice de sortie (16) et le siège de valve (19), et le disque d'étanchéité (40, 50) est monté à l'intérieur de la chambre de valve (18).

4. Valve PEEP telle que revendiquée à la Revendication 3, où la chambre de valve (18) est essentiellement cylindrique avec l'orifice d'entrée (14) à une extrémité de la chambre de valve (18), l'autre extrémité de la chambre de valve (18) étant fermée, et l'orifice de sortie (16) bifurquant perpendiculairement à partir du côté de la chambre de valve (18).

5. Valve PEEP telle que revendiquée à la Revendication 4, où l'extrémité fermée de la chambre de valve (18) inclut un support pour le disque d'étanchéité (40, 50).

6. Valve PEEP telle que revendiquée à la Revendication 5, où l'extrémité fermée de la chambre de valve (18) est formée par une fermeture (20) qui est formée intégralement avec au moins une partie du support pour le disque d'étanchéité (40,50) et qui est reçue étroitement à l'intérieur du boîtier (12).

7. Valve PEEP telle que revendiquée dans une quelconque revendication précédente, où le support pour le disque d'étanchéité (40, 50) comprend un canal de section transversale constante qui a une extrémité ouverte pour recevoir un arbre (30) de manière à ce que l'arbre (30) puisse se déplacer en va-et-vient à l'intérieur du canal, l'extrémité de l'arbre (30) qui dépasse de l'extrémité ouverte du canal étant fixée au disque d'étanchéité (40, 50).

8. Valve PEEP telle que revendiquée à la Revendication 7, où l'arbre (30) a une tige (32) s'étirant de manière coaxiale à partir de l'extrémité de l'arbre (30) qui est fixée à l'intérieur d'un alésage central du disque d'étanchéité (40, 50).

9. Valve PEEP telle que revendiquée dans une quelconque revendication précédente, où la valve inclut des moyens élastiques pour solliciter le disque d'étanchéité (40, 50) en mise en prise avec le siège de valve (19).

10. Valve PEEP telle que revendiquée à la Revendication 9, où les moyens élastiques sont un ressort à force constante.

11. Valve PEEP telle que revendiquée dans une quelconque revendication précédente, où le siège de valve (19) est une formation surélevée sur la surface intérieure du boîtier (12).

12. Valve PEEP telle que revendiquée à la Revendication 11, où le siège de valve (19) est généralement de forme annulaire et définit une ouverture centrale, et le disque d'étanchéité (40, 50) vient en prise avec le siège de valve (19) de manière à sceller l'ouverture définie par le siège de valve (19).

13. Valve PEEP telle que revendiquée dans une quelconque revendication précédente, où la fermeture (44, 54) recouvre la surface du disque d'étanchéité (40, 50) qui est orientée dans la direction de l'orifice d'entrée (14).

14. Valve PEEP telle que revendiquée dans une quelconque revendication précédente, où la fermeture (44, 54) est formée d'un matériau élastomère.

15. Valve PEEP telle que revendiquée dans une quelconque revendication précédente, où la fermeture (44, 54) est fixée au disque d'étanchéité (40, 50) à un bord de la fermeture (44, 54) et au-delà de la périphérie de l'ouverture que la fermeture (44, 54) occlut.

16. Valve PEEP telle que revendiquée dans une quelconque revendication précédente, où le disque d'étanchéité (40, 50) inclut deux ou plusieurs ouvertures (42, 52).

17. Valve PEEP telle que revendiquée à la Revendication 16, où le disque d'étanchéité (40, 50) inclut six ouvertures (42, 52) ou plus.

18. Valve PEEP telle que revendiquée à la Revendication 16 ou Revendication 17, où les ouvertures (42, 52) sont disposées en groupes, chaque groupe d'ouvertures (42, 52) coopérant avec l'une de deux ou plusieurs fermetures (44, 54).

19. Valve PEEP telle que revendiquée à la Revendication 18, où chaque fermeture (44, 54) occlut deux ou plusieurs ouvertures (42, 52).

20. Valve PEEP telle que revendiquée à la Revendication 19, où le disque d'étanchéité (40, 50) est muni de deux groupes diamétralement opposés d'ouvertures (42, 52), et chaque groupe d'ouvertures (42, 52) est occlus par une seule fermeture (44, 54), de telle manière à ce que la valve PEEP ait une paire de fermetures diamétralement opposées (44, 54).

21. Valve PEEP telle que revendiquée à la Revendication 20, où chacune des ouvertures diamétralement opposées (44, 54) est généralement de forme elliptique.

22. Valve PEEP telle que revendiquée dans une quelconque des Revendications 18 à 21, où les deux ou plusieurs fermetures (44, 54) forment une partie d'un composant à fermeture unique qui est fixé au disque d'étanchéité (40, 50).
